Europäisches Patentamt

European Patent Office

Office européen des brevets

(11) Veröffentlichungsnummer: **0 073 404**
**B1**

(12) **EUROPÄISCHE PATENTSCHRIFT**

(45) Veröffentlichungstag der Patentschrift:
27.12.84

(21) Anmeldenummer: **82107458.0**

(22) Anmeldetag: **17.08.82**

(51) Int. Cl.³: **C 07 C 119/02**

(54) Verfahren zur Herstellung von Aryl-Isocyanid-dichloriden.

(30) Priorität: **28.08.81 DE 3134134**

(43) Veröffentlichungstag der Anmeldung:
**09.03.83 Patentblatt 83/10**

(45) Bekanntmachung des Hinweises auf die Patenterteilung:
**27.12.84 Patentblatt 84/52**

(84) Benannte Vertragsstaaten:
**CH DE FR GB LI**

(56) Entgegenhaltungen:
**DE - C - 1 094 737**

**ANGEWANDTE CHEMIE, Band 79, Nr. 15, 21. Juli 1967, E. KÜHLE et al. "Isocyaniddihalogenid-Synthesen", Selten 663 bis 680**

(73) Patentinhaber: **CASSELLA Aktiengesellschaft, Hanauer Landstrasse 526, D-6000 Frankfurt am Main 61 (DE)**

(72) Erfinder: **Ferchland, Dieter, Bergstrasse 17, D-6233 Kelkheim/Ts. (DE)**
Erfinder: **Kussmaul, Ulrich, Dr., Ulmenweg 1, D-6369 Niederdorfelden (DE)**
Erfinder: **Langer, Manfred, Dr., Birsteiner Strasse 47, D-6000 Frankfurt am Main 61 (DE)**
Erfinder: **Müller, Rolf, Dr., Dornbachweg 3, D-6367 Karben 4 (DE)**

(74) Vertreter: **Urbach, Hans-Georg, Dr. et al, Hanauer Landstrasse 526, D-6000 Frankfurt am Main 61 (DE)**

ACTORUM AG

## Beschreibung

Die Verfahren zur Herstellung von Arylisocyaniddichloriden sind z.B. zusammenfassend in „Angew. Chem.", 79 (1967), S. 663-680, beschrieben worden. Für eine technische Herstellung von Arylisocyaniddichloriden kommen im wesentlichen die Chlorierung von Isothiocyanaten, die Chloraddition an Isonitrile und die Chlorierung von N-Arylformamiden in Gegenwart von Thionylchlorid in Betracht.

Bei der Chlorierung von Arylisothiocyanaten (Senfölen) entsteht neben dem gewünschten Arylisocyaniddichlorid Schwefeldichlorid als unerwünschtes Nebenprodukt. Bei der Anlagerung von Chlor an Arylisocyanide müssen die giftigen, übelriechenden und instabilen Arylisocyanide verarbeitet werden. Die Chlorierung von Formanilid in Anwesenheit von Chloroform und Thionylchlorid (vgl. „J. Am. Chem. Soc.", 44 (1922), 2896 ff.) liefert ein Produktgemisch und nur schlechte Ausbeuten an Phenylisocyaniddichlorid. Unter bewusstem Verzicht auf organische Lösungsmittel werden bei der Chlorierung von N-Arylformamiden in überschüssigem Thionylchlorid (vgl. DE-PS Nr. 1094737) in der Regel gute Ausbeuten erhalten. Dieses bekannte Verfahren erfordert jedoch den Einsatz von isolierten N-Arylformamiden, die demgemäss zunächst in getrennten Verfahren hergestellt, isoliert und getrocknet werden müssen. Ein zusätzlicher technischer Aufwand ist zum Transport und zur Dosierung der bei Raumtemperatur festen N-Arylformamide erforderlich.

Überraschenderweise wurde jetzt gefunden, dass eine getrennte Herstellung und Isolierung der N-Arylformamide nicht notwendig ist, vielmehr können diese in einem geeigneten Lösungsmittel durch Formylierung von Arylaminen hergestellt und anschliessend ohne Isolierung mit Thionylchlorid und einem Chlorierungsmittel zu den Arylisocyaniddichloriden umgesetzt werden. Dabei werden bei hohen Reinheiten Ausbeuten an Arylisocyaniddichloriden erzielt, die den erzielbaren besten Ausbeuten des Standes der Technik gleichen oder diese sogar übertreffen. Dies war aufgrund des bisherigen Kenntnisstandes, insbesondere des Vorurteils gegen die Verwendung organischer Lösungsmittel bei der Chlorierung der N-Arylformamide, nicht zu erwarten.

Nach dem erfindungsgemässen Verfahren wird zunächst ein gegebenenfalls ein- oder mehrfach kernsubstituiertes Arylamin mit 6 bis 12 C-Atomen, vorzugsweise ein ein-, zwei- oder dreifach kernsubstituiertes Anilin, in einem geeigneten inerten Lösungsmittel in an sich bekannter Weise formyliert und in das entsprechende N-Arylformamid überführt. Die Formylierung kann mit allen Agenzien durchgeführt werden, die eine aromatische Aminogruppe zu formylieren vermögen, wie z.B. mit Ameisensäure, Ameisensäureessigsäureanhydrid, Ameisensäureester, insbesondere Ameisensäuremethylester (vgl. „Ullmanns Encyklopädie der techn. Chemie", 4. Aufl., Bd. 11 (1976), 708), oder Formamide, wie Formamid selbst oder Dimethylformamid (vgl. „J. Org. Chem.", 26 (1961), 2563 ff.). Die Formylierung mit Ameisensäure ist im Rahmen der vorliegenden Erfindung bevorzugt.

Die Formylierung wird in einem geeigneten Lösungsmittel oder Lösungsmittelgemisch durchgeführt. Das verwendete Lösungsmittel soll in allen Reaktionsstufen des erfindungsgemässen Verfahrens, d.h. sowohl bei der Formylierung des Arylamins als auch bei der nachfolgenden Chlorierung des N-Arylformamids, gegenüber Ausgangs-, Zwischen- und Endprodukten inert sein. Toluol, das oft als Lösungsmittel für Formylierungen verwendet wird, ist als Lösungsmittel beim erfindungsgemässen Verfahren wenig geeignet, da es mit dem Chlorierungsmittel reagieren kann. Das benutzte Lösungsmittel sollte einen Siedepunkt besitzen, der sich genügend weit von den Siedepunkten des Arylisocyaniddichlorids und des Thionylchlorids unterscheidet, um eine destillative Trennung des Lösungsmittels von diesen Verbindungen zu ermöglichen. Bevorzugt werden solche Lösungsmittel, deren Siedepunkte sich um mindestens 30°C von den Siedepunkten des Arylisocyaniddichlorids und des Thionylchlorids unterscheiden, wie dies z.B. normalerweise bei höheren acyclischen und cyclischen Alkanen der Fall ist, die mindestens 8 C-Atome im Molekül besitzen. Geeignete Vertreter sind z.B. n-Octan, n-Nonan, n-Decan, n-Dodecan, n-Pentadecan, 2,2,3-Trimethylpentan, 2,2,3-Trimethylhexan, 1,1-Dimethylcyclohexan. Diese Alkane können gegebenenfalls auch in Form ihrer technischen Gemische eingesetzt werden. Bei Alkanen mit mehr als 20 C-Atomen wird in der Regel der Schmelzpunkt zu hoch. Bevorzugt sind ferner polare Lösungsmittel, insbesondere solche, die eine Polarität gleich oder grösser der von Chlorbenzol besitzen, d.h. deren Dipolmoment gleich oder grösser des von Chlorbenzol ist. Ganz besonders bevorzugte Lösungsmittel sind solche, die mehrere oder insbesondere alle der vorgenannten bevorzugten Eigenschaften besitzen, wie z.B. halogenierte alicyclische Alkane mit z.B. mindestens 2 C-Atomen, mit in der Regel 2 bis 10 C-Atomen, oder halogenierte Cycloalkane mit 5 bis 10 C-Atomen. Besonders bevorzugt sind höher halogenierte Alkane, d.h. Alkane, die 2 und mehr Halogenatome im Molekül besitzen, sowie einfach oder höher chlorierte und/oder nitrierte Benzole. Die Halogenatome der halogenierten Alkane und Benzole sind dabei vorzugsweise Chloratome. Beispiele für geeignete halogenierte Alkane sind die verschiedenen Chlorpentane, 1-Chlorhexan, Chlorcyclohexan. Beispiele für geeignete höher halogenierte Alkane sind 1,1,2-Trichlorethan, 1,2,3-Trichlorpropan, 1,1,2,2-Tetrachlorethan, 1,1,2,2-Tetrachlorpropan, Hexachlorethan, Pentachlorethan, die verschiedenen Tetrachlorpentane. Beispiele für einfach und höher halogenierte und/oder nitrierte Benzole sind Chlorbenzol, Brombenzol, o-Dichlorbenzol, m-Dichlorbenzol, p-Dichlorbenzol, isomere Trichlorbenzole, Benzotrichlorid, 2-Chlorbenzotrifluorid, 3-Chlorbenzotrifluorid, 4-Chlorbenzotrifluorid, Nitrobenzol, 2-Chlornitrobenzol, 3-Chlornitrobenzol, 4-Chlornitrobenzol. Besonders bevorzugt sind Chlorbenzol, o-Dichlorbenzol und Nitrobenzol.

Um bei der Formylierung eine vollständige Umsetzung zu erreichen, wird das Formylierungsmittel, vorzugsweise die Ameisensäure, im molaren Überschuss eingesetzt. Dieser Überschuss beträgt normalerweise und in Abhängigkeit von der Konzentration der eingesetzten Ameisensäure das 1,1- bis 8fache, vorzugsweise das 1,5- bis 4fache, der theoretisch benötigten Menge. Die Formylierung wird normalerweise bei erhöhter Temperatur, vorzugsweise bei der Rückflusstemperatur, des benutzten Lösungsmittels, bei Atmosphärendruck, erhöhtem oder vermindertem Druck durchgeführt. Während oder im Anschluss an die Reaktion werden überschüssiges Formylierungsmittel und die bei der Formylierung entstehenden Nebenprodukte (Wasser bei der Formylierung mit Ameisensäure und Methanol bei der Formylierung mit Ameisensäuremethylester) abdestilliert. In vielen Fällen kann dabei, wie z.B. bei Verwendung von Chlorbenzol oder o-Dichlorbenzol als Lösungsmittel, durch azeotrope Destillation des Reaktionswassers oder gegebenenfalls überschüssiger Ameisensäure ein praktisch quantitativer Umsatz zu den N-Arylformamiden erreicht werden. Die N-Arylformamide fallen als Lösung oder als Suspension in dem verwendeten Lösungsmittel an.

Als Arylamine für die Formylierung sind z.B. geeignet: Anilin; im Kern mono-, di- oder trisubstituierte Aniline, wobei als Substituenten z.B. in Betracht kommen: Halogen, wie z.B. Fluor, Chlor, Brom, Nitro, Trifluormethyl, Alkyl mit z.B. 1 bis 4, vorzugsweise 1 oder 2, C-Atomen, beispielsweise n-Propyl, Isopropyl, n-Butyl, Isobutyl, tert.-Butyl, vorzugsweise Methyl oder Ethyl, Alkoxy mit z.B. 1 bis 4, vorzugsweise 1 oder 2, C-Atomen, wie z.B. n-Butoxy, Isopropoxy, vorzugsweise Methoxy oder Ethoxy; Phenyl; Halogenphenyl, wie z.B. 4-Chlorphenyl; Phenylazo; Phenyloxy; Halogenphenyloxy, wie z.B. 4-Chlorphenyloxy; Benzoyl; Halogenbenzoyl, wie z.B. 4-Chlorbenzoyl; Phenylsulfonyl; Halogenphenylsulfonyl, wie z.B. 4-Chlorphenylsulfonyl; Alkoxycarbonyl, z.B. mit 2 bis 5 C-Atomen, wie z.B. Methoxycarbonyl; Halogencarbonyl, wie z.B. Chlorcarbonyl. Als Arylamine kommen auch Naphthylamine, wie z.B. das 1-Naphthylamin, und im Kern substituierte Naphthylamine in Betracht. Als Ausgangsarylamine sind z.B. folgende Verbindungen zu nennen: Anilin; Monohalogenaniline, wie z.B. 2-, 3- oder 4-Chloranilin, 2-, 3- oder 4-Bromanilin, 2-, 3- oder 4-Fluoranilin; Mononitraniline, wie z.B. 3- oder 4-Nitroanilin, Dihalogenaniline, wie z.B. 2,3-, 2,4-, 2,5-, 2,6-, 3,4-, 3,5-Dichlor- oder Dibromanilin; Halogennitraniline, wie z.B. 2-Chlor-4-nitranilin, 4-Chlor-3-nitranilin; Dihalogennitraniline, wie z.B. 2,5-Dichlor-4-nitranilin; Halogenalkylaniline, wie z.B. 2-Methyl-4-chloranilin, 2-Chlor-6-methylanilin; Dihalogenalkylaniline wie z.B. 2,4-Dichlor-5-methyl- oder -5-ethylanilin, 2,5-Dichlor-4-ethylanilin; Alkoxyhalogenaniline, wie z.B. 2-Ethoxy-4-chloranilin; Alkylnitraniline, wie z.B. 2-Methyl-5-nitranilin, 4-Methyl-3-nitranilin, 4-Ethyl-3-nitranilin; Trihalogenaniline, wie z.B. 2,4,5-Trihalogenanilin; Trifluormethylaniline, wie z.B. 4-Trifluormethylanilin; Halogentrifluormethylaniline, wie z.B. 2-Chlor-5-trifluormethylanilin, 2-Trifluormethyl-4-chloranilin; Alkylaniline; Dialkylaniline, wie z.B. 2,4-Dimethyl- oder -Diethylanilin, 2,6-Diethyl- oder -Diisopropylanilin; Trialkylaniline, wie z.B. 2,4,6-Trimethylanilin, 2,4,6-Triethylanilin, 2,6-Diethyl-4-methylanilin; Alkoxycarbonylaniline, wie z.B. 4-Methoxycarbonylanilin, 4-Ethoxycarbonylanilin; 4-Chlorcarbonylanilin; 4-Aminoazobenzol; 4,4'-Dichlor-2-aminodiphenylether; 4-Aminobenzophenon; 4-Aminodiphenylsulfon; 4-Phenylanilin; 1-Naphthylamin. Die Ausbeuten an Arylisocyaniddichloriden sind dann hoch, wenn eine Chlorierung im Kern nicht möglich ist und wenn der Kern nicht einen oder mehrere Substituenten trägt, die eine nukleophile aromatische Substitution stark erleichtern.

Die in dem Lösungsmittel als Lösung oder Dispersion vorliegenden N-Arylformamide werden nun in Form der Lösung oder Suspension, zweckmässigerweise in dem Gefäss ihrer Herstellung, in Gegenwart von Thionylchlorid mit einem Chlorierungsmittel chloriert. Als Chlorierungsmittel kommen z.B. elementares Chlor oder Chlor abgebende Substanzen, wegen der leichten Dosierbarkeit insbesondere Sulfurylchlorid, in Betracht. Pro Mol N-Arylformamid werden mindestens 1 mol Chlorierungsmittel und 1 mol Thionylchlorid eingesetzt. Normalerweise werden pro Mol N-Arylformamid 1 bis 2,5 mol Chlorierungsmittel eingesetzt. Die Verwendung eines noch höheren molaren Überschusses an Chlorierungsmittel ist zwar möglich, bringt aber keine Vorteile. Besonders gute Ausbeuten (bis zu 90%) und hohe Reinheiten (bis zu 98% und mehr) werden erhalten, wenn das Molverhältnis Thionylchlorid/Chlorierungsmittel grösser als 1:1 ist und z.B. (4 bis 10):1 beträgt. Noch höhere Molverhältnisse zwischen Thionylchlorid und Chlorierungsmittel sind möglich, bringen aber keine Vorteile. Nach den vorstehenden Angaben beträgt das Molverhältnis N-Arylformamid (bzw. Arylamin, da mit einer vollständigen Formylierung zu rechnen ist)/Chlorierungsmittel/Thionylchlorid normalerweise 1: (1 bis 2,5): (1 bis 25), vorzugsweise 1: (1 bis 2,5): (4 bis 25).

Besonders bequem ist es, die Chlorierung der erhaltenen Lösung oder Suspension des N-Arylformamids in dem Gefäss vorzunehmen, in dem diese Lösung oder Suspension erhalten worden ist. Das erfindungsgemässe Verfahren gestattet dadurch, die Herstellung der Arylisocyaniddichloride ausgehend von Arylaminen in einem einzigen Gefäss durchzuführen (Eintopfverfahren). Bei diesem Eintopfverfahren kann dann bei der für die Isocyaniddichloridbildung erforderlichen Temperatur direkt die gesamte Einsatzmenge Thionylchlorid und Halogenierungsmittel auf einmal zugesetzt werden. Erstaunlicherweise bedeutet diese Verfahrensweise kein Sicherheitsrisiko, da die zunächst unlöslichen Intermediärprodukte erst allmählich mit dem Reaktionsfortschritt in Lösung gehen und die Reaktionswärme ohne externe Kühlung durch die entweichenden Reaktionsgase abgeführt wird. Selbstverständlich können aber auch Thionylchlorid und/oder Halogenierungsmittel im Verlauf der Reaktion zudosiert werden. Es ist auch möglich, ein Gemisch aus Thionylchlorid und Halogenierungsmittel vorzulegen und die Lösung oder Suspension des N-Arylformamids zuzudosieren, was ebenfalls gute und in einigen Fällen sogar leicht verbesserte Ausbeuten an Arylisocyaniddichloriden ergibt.

Die Chlorierung wird bei Temperaturen von 0 bis 80°C durchgeführt; besonders günstige Ergebnisse erzielt man bei Temperaturen von 10 bis 60°C.

Durch einfache Destillation, vorzugsweise unter vermindertem Druck, können nach Beendigung der Chlorierung überschüssiges Thionylchlorid und gegebenenfalls Chlorierungsmittel sowie das organische Lösungsmittel regeneriert und die Arylisocyaniddichloride isoliert werden.

*Beispiel 1:*

162 g (1 mol) 2,4-Dichloranilin werden in 220 ml o-Dichlorbenzol gelöst und durch Kochen mit 135 ml 85 gew.%iger Ameisensäure (3 mol) quantitativ zu N-2,4-Dichlorphenylformamid umgesetzt, wobei das Reaktionswasser und überschüssige Ameisensäure azeotrop 4 h lang bei Atmosphärendruck und anschliessend 4 h bei einem Druck von 160 mbar abdestilliert werden. Bei 22°C gibt man zu der Suspension 86 ml (1 mol) Sulfurylchlorid und 585 ml (8,1 mol) Thionylchlorid auf einmal zu. Nach ca. 1 h beginnt eine deutliche Abspaltung von Chlorwasserstoff und Schwefeldioxid, die in einer 2stufigen Absorption in Wasser bzw. Natronlauge aufgenommen werden. Im Zeitraum von 2 bis 8 h nach der Zugabe des Sulfurylchlorids und des Thionylchlorids wird die maximale Gasentwicklung beobachtet (180 Blasen/min). Die Temperatur bleibt ohne externe Kühlung bei 20 bis 24°C. Dann wird langsam bis zum Rückfluss erwärmt und überschüssiges Thionylchlorid und o-Dichlorbenzol (im Wasserstrahlvakuum) abdestilliert. Man erhält durch Destillation 217,9 g 2,4-Dichlorphenylisocyaniddichlorid, Siedepunkt 124 bis 128°C bei 18 mbar, gaschromatographische Reinheit > 98%, entsprechend einer Ausbeute von 89,7%, bezogen auf 2,4-Dichloranilin.

*Beispiel 2:*

Die nach Beispiel 1 hergestellte Suspension von N-(2,4-Dichlorphenyl)formamid wird in 6 h bei 40°C zu einem Gemisch von 85 ml Sulfurylchlorid und 585 ml Thionylchlorid zudosiert, wobei wie im Beispiel 1 eine kontinuierliche Gasentwicklung (ca. 160 Blasen/min) beobachtet wird. Die Aufarbeitung analog Beispiel 1 ergibt 218,8 g 2,4-Dichlorphenylisocyaniddichlorid, gaschromatographische Reinheit > 98%, entsprechend 90,1% Ausbeute, bezogen auf 2,4-Dichloranilin.

*Beispiel 3:*

127,6 g (1 mol) 2-Chloranilin werden in 250 ml Chlorbenzol wie im Beispiel 1 im Eintopfverfahren umgesetzt. Man erhält 183,5 g 2-Chlorphenylisocyaniddichlorid, Siedepunkt 104 bis 106°C/13,3 mbar, gaschromatographische Reinheit > 98%, entsprechend einer Ausbeute von 88%, bezogen auf 2-Chloranilin.

*Beispiel 4:*

Die nach Beispiel 1 hergestellte Suspension von N-(2,4-Dichlorphenyl)formamid wird analog Beispiel 2 weiterverarbeitet, statt Sulfurylchlorid wird jedoch ein ständiger Chlorgasstrom eingeleitet, so dass insgesamt mindestens 1,1 mol Chlor zugegeben wird. Man erhält 207,5 g 2,4-Dichlorphenylisocyaniddichlorid, Reinheit > 98%, entsprechend einer Ausbeute von 85,4%.

*Beispiel 5:*

93,1 g (1 mol) Anilin werden in 200 ml Chlorbenzol mit 740,1 g (10 mol) Ameisensäureethylester versetzt und in einem Autoklaven 3 h bei 110°C erhitzt. Danach werden Ethanol und überschüssiger Ameisensäureethylester abdestilliert. Der Rückstand wird analog Beispiel 1 bei 10°C mit 1,0 mol Sulfurylchlorid und 8,1 mol Thionylchlorid versetzt. Nach Abdestillieren von überschüssigem Thionylchlorid und des Chlorbenzols erhält man 83,5 g Phenylisocyaniddichlorid, Siedepunkt 95 bis 98°C bei 18 mbar, entsprechend einer Ausbeute von 48,0%, bezogen auf Anilin.

Analog den Beispielen 1 bis 5 lassen sich die nachstehend angegebenen Isocyaniddichloride in den angegebenen Lösungsmitteln im Eintopfverfahren herstellen.

| Beispiel | Isocyaniddichlorid | Lösungsmittel | Ausbeute (%) |
|---|---|---|---|
| 6 | 4-Chlorphenyl- | 1,1,2,2-Tetrachlorethan | 86 |
| 7 | 4-Nitrophenyl- | Nitrobenzol | 89 |
| 8 | 2,4,5-Trichlorphenyl- | o-Dichlorbenzol | 90 |
| 9 | 2-Chlor-6-methylphenyl- | Chlorbenzol | 93 |
| 10 | 2-Chlor-5-trifluormethylphenyl- | Chlorbenzol | 75 |
| 11 | 4-Methoxycarbonylphenyl- | o-Dichlorbenzol | 90 |
| 12 | Benzophenon-4- | o-Dichlorbenzol | 75 |
| 13 | 4-Bromphenyl- | Chlorbenzol | 74 |

Als Formylierungsmittel kann bei den Beispielen 6 bis 12 85- bis 100%ige Ameisensäure oder Ameisensäureessigsäureanhydrid verwendet werden, bei den Beispielen 6, 9 und 13 auch Ameisensäuremethylester.

## Patentansprüche

1. Verfahren zur Herstellung von Arylisocyaniddichloriden durch Chlorierung von N-Arylformamiden in Gegenwart von Thionylchlorid, dadurch gekennzeichnet, dass Arylamine in einem inerten Lösungsmittel mit einem Siedepunkt, der sich genügend weit von den Siedepunkten der entstehenden Arylisocyaniddichloride und des Thionylchlorids unterscheidet, um eine destillative Trennung zu ermöglichen, in an sich bekannter Weise durch Formylierung in N-Arylformamide überführt werden und dass, nach der Entfernung des überschüssigen Formylierungsmittels und der bei der Formylierung entstehenden Nebenprodukte, die in Form einer Lösung oder Suspension in dem Lösungsmittel vorliegenden N-Arylformamide in Gegenwart von Thionylchlorid mit einem Chlorierungsmittel bei Temperaturen von 0 bis 80°C chloriert und danach die Arylisocyaniddichloride durch Destillation isoliert werden.

2. Verfahren nach Anspruch 1, dadurch gekennzeichnet, dass als Arylamine Aniline verwendet werden, die im Kern gegebenenfalls ein-, zwei- oder dreifach substituiert sind.

3. Verfahren nach den Ansprüchen 1 und/oder 2, dadurch gekennzeichnet, dass als Formylierungsmittel Ameisensäure verwendet wird.

4. Verfahren nach einem oder mehreren der Ansprüche 1 bis 3, dadurch gekennzeichnet, dass als inertes Lösungsmittel ein polares Lösungsmittel, insbesondere ein solches mit einem Dipolmoment gleich oder grösser des von Chlorbenzol, verwendet wird.

5. Verfahren nach einem oder mehreren der Ansprüche 1 bis 4, dadurch gekennzeichnet, dass als inertes Lösungsmittel ein halogeniertes Alkan, ein halogeniertes oder nitriertes Benzol, insbesondere Chlorbenzol, o-Dichlorbenzol oder Nitrobenzol, oder ein Gemisch dieser Lösungsmittel verwendet wird.

6. Verfahren nach einem oder mehreren der Ansprüche 1 bis 5, dadurch gekennzeichnet, dass als Chlorierungsmittel elementares Chlor oder vorzugsweise Sulfurylchlorid verwendet wird.

7. Verfahren nach einem oder mehreren der Ansprüche 1 bis 6, dadurch gekennzeichnet, dass das Molverhältnis Arylamin/Chlorierungsmittel/Thionylchlorid 1 : (1 bis 2,5) : (1 bis 25), vorzugsweise 1 : (1 bis 2,5) : (4 bis 25), beträgt.

8. Verfahren nach einem oder mehreren der Ansprüche 1 bis 7, dadurch gekennzeichnet, dass die Chlorierung bei Temperaturen von 10 bis 60°C durchgeführt wird.

9. Verfahren nach einem oder mehreren der Ansprüche 1 bis 8, dadurch gekennzeichnet, dass als Arylamin 2,4-Dichloranilin eingesetzt wird.

10. Verfahren nach einem oder mehreren der Ansprüche 1 bis 9, dadurch gekennzeichnet, dass es als Eintopfverfahren durchgeführt wird.

## Claims

1. Process for the preparation of aryl isocyanide-dichlorides by chlorinating N-arylformamides in the presence of thionyl chloride, characterised in that arylamines are converted into N-arylformamides by formylation in a manner which is in itself known in an inert solvent having a boiling-point which is different from the boiling-points of the aryl isocyanide-dichlorides formed and of the thionyl chloride to such an extent that a separation by distillation is possible, and, after removing the excess formylating agent and the by-products formed in the formylation, the N-arylformamides which are present in the form of a solution or suspension in the solvent are chlorinated by means of a chlorinating agent at temperatures between 0 and 80°C in the presence of thionyl chloride, and that the aryl isocyanide-dichlorides are then isolated by distillation.

2. Process according to Claim 1, characterised in that the arylamines used are anilines which are optionally mono-, di- or trisubstituted in the nucleus.

3. Process according to Claims 1 and/or 2, characterised in that formic acid is used as the formylating agent.

4. Process according to one or more of Claims 1 to 3, characterised in that the inert solvent used is a polar solvent, in particular a polar solvent having a dipole moment equal to or greater than that of chlorobenzene.

5. Process according to one or more of Claims 1 to 4, characterised in that the inert solvent used is a halogenated alkane, a halogenated or nitrated benzene, in particular chlorobenzene, o-dichlorobenzene or nitrobenzene, or a mixture of these solvents.

6. Process according to one or more of Claims 1 to 5, characterised in that elementary chlorine or, preferably, sulphuryl chloride is used as the chlorinating agent.

7. Process according to one or more of Claims 1 to 6, characterised in that the molar ratio arylamine/chorinating agent/thionylchloride is 1 : (1 to 2.5) : (1 to 25), preferably 1 : (1 to 2.5) : (4 to 25).

8. Process according to one or more of Claims 1 to 7, characterised in that the chlorination is carried out at temperatures of 10 to 60°C.

9. Process according to one or more of Claims 1 to 8, characterised in that the arylamine employed is 2,4-dichloroaniline.

10. Process according to one or more of Claims 1 to 9, characterised in that it is carried out as a one-pot process.

## Revendications

1. Procédé de préparation de dichloro-isocyanures d'aryles par chloration de N-arylformamides en présence de chlorure de thionyle, procédé caractérisé en ce qu'on transforme des arylamines en N-arylformamides de manière connue, par formylation, en opérant dans un solvant inerte dont le point d'ébullition est suffisamment distant des points d'ébullition des dichloro-isocyanures d'aryles formés et du chlorure de thionyle pour qu'une séparation par distillation soit possible, puis, après avoir éliminé l'excès d'agent de formylation et les produits secondaires engendrés lors de la formylation, on chlore les N-arylformamides, qui sont sous la forme d'une solution ou d'une suspension dans le solvant, en présence de chlorure de thionyle, au moyen d'un agent de chloration, à des températures de 0 à 80°C, après quoi on isole les dichloro-isocyanures d'aryles par distillation.

2. Procédé selon la revendication 1, caractérisé en ce qu'on utilise, comme arylamines, des anilines dont le noyau porte éventuellement un, deux ou trois substituants.

3. Procédé selon l'une des revendications 1 et/ou 2, caractérisé en ce qu'on utilise, comme agent de formylation, l'acide formique.

4. Procédé selon l'une des revendications 1 à 3, caractérisé en ce qu'on utilise, comme solvant inerte, un solvant polaire, plus particulièrement un solvant polaire ayant un moment dipolaire égal ou supérieur à celui du chlorobenzène.

5. Procédé selon l'une des revendications 1 à 4, caractérisé en ce qu'on utilise, comme solvant inerte, un alcane halogéné, un benzène halogéné ou nitré, plus spécialement le chlorobenzène, l'o-dichlorobenzène ou le nitrobenzène, ou un mélange de ces solvants.

6. Procédé selon l'une des revendications 1 à 5, caractérisé en ce qu'on utilise, comme agent de chloration, le chlore élémentaire ou, mieux, le chlorure de sulfuryle.

7. Procédé selon l'une des revendications 1 à 6, caractérisé en ce que le rapport molaire arylamine/agent de chloration/chlorure de thionyle est de 1 : (1 à 2,5) : (1 à 25), de préférence de 1 : (1 à 2,5) : (4 à 25).

8. Procédé selon l'une des revendications 1 à 7, caractérisé en ce que la chloration est effectuée à des températures de 10 à 60°C.

9. Procédé selon l'une des revendications 1 à 8, caractérisé en ce qu'on utilise, comme arylamine, la dichloro-2,4-aniline.

10. Procédé selon l'une des revendications 1 à 9, caractérisé en ce qu'on opère en un seul récipient.